# EUROPÄISCHE PATENTSCHRIFT

(11) **EP 3 244 962 B1**
(45) Veröffentlichungstag und Bekanntmachung des Hinweises auf die Patenterteilung: **17.10.2018**
(21) Anmeldenummer: 16700602.2
(22) Anmeldetag: 14.01.2016
(51) Int. Cl.: A61M 39/10, A61M 39/12, F16L 33/20, F16L 33/22, F16L 33/24

(54) **VERBINDUNGSVORRICHTUNG FÜR EIN MEDIZINISCHES FLUIDLEITUNGSSYSTEM**
CONNECTION DEVICE FOR A MEDICAL FLUID CONDUIT SYSTEM
DISPOSITIF DE RACCORDEMENT POUR SYSTÈME DE LIGNE DE FLUIDE MÉDICAL

(30) Priorität: 16.01.2015 DE 102015200613
(43) Veröffentlichungstag der Anmeldung: 22.11.2017
(73) Patentinhaber: B. Braun Melsungen AG, 34212 Melsungen (DE)
(72) Erfinder: FUCHS, Jürgen, 34308 Bad Emstal (DE); HARTUNG, Jürgen, 37235 Hessisch Lichtenau (DE)
(74) Vertreter: Patentanwälte Ruff, Wilhelm, Beier, Dauster & Partner mbB
(86) Internationale Anmeldenummer: PCT/EP2016/050635
(87) Internationale Veröffentlichungsnummer: WO 2016/113337

(56) Entgegenhaltungen:
- WO-A1-2009/055949
- WO-A1-2010/028052
- WO-A1-2010/088512
- DE-A1- 4 129 781
- DE-A1-102008 008 332
- DE-A1-102015 108 596
- DE-C- 570 905
- US-B1- 6 641 177

## Beschreibung

Die Erfindung betrifft eine Verbindungsvorrichtung für ein medizinisches Fluidleitungssystem mit einem Verbindungskörper, der einen Schlauchanschlussstutzen sowie einen Verbindungsanschluss zur lösbaren Befestigung einer weiteren Fluidleitungs- oder Speicherkomponente aufweist, wobei der Verbindungskörper von einer Sicherungshülse koaxial umgeben ist und wobei in betriebsfertigem Zustand eine Schlauchleitung mit dem Schlauchanschlussstutzen verbunden ist. Die Erfindung betrifft zudem ein medizinisches Fluidleitungssystem mit einer Schlauchleitung sowie mit einer Verbindungsvorrichtung zur Befestigung einer weiteren Fluidleitungs- oder -speicherkomponente, wobei die Verbindungsvorrichtung einen Verbindungskörper aufweist, mit dem ein Schlauchabschnitt der Schlauchleitung verbunden ist.

Derartige medizinische Fluidleitungssysteme sind allgemein bekannt. Diese weisen üblicherweise eine Schlauchleitung auf, deren endseitiger Schlauchabschnitt mit einem Schlauchanschlussstutzen eines Verbindungskörpers verklebt ist. Der Verbindungskörper weist an seinem dem Schlauchanschlussstutzen gegenüberliegenden Stirnendbereich einen Verbindungsanschluss zur Befestigung einer weiteren Fluidleitungskomponente oder Fluidspeicherkomponente auf. Der Verbindungskörper ist von einer Sicherungshülse umgeben, die mit einem weiblichen Luer-Lock-Gewinde versehen ist, um eine Verbindung mit einem männlichen Luer-Lock-Gewinde der entsprechend anzuschließenden Fluidleitungskomponente oder Fluidspeicherkomponente einzugehen. Dadurch, dass der Schlauchabschnitt mit dem Verbindungskörper verklebt ist, muss sowohl das Schlauchmaterial des Schlauchabschnitts als auch das Material des Verbindungskörpers geeignete physikalische und chemische Eigenschaften aufweisen, um zusammen mit einem entsprechenden Klebstoff eine stabile Klebeverbindung zu gewährleisten.
Ein Verbindungsvorrichtung gemäss dem Oberbegriff von Anspruch 1 ist aus dem Dokument DE 570 905 C bekannt.

Aufgabe der Erfindung ist es, eine Verbindungsvorrichtung und ein medizinisches Fluidleitungssystem der eingangs genannten Art zu schaffen, die eine einfache Handhabung und verbesserte Einsatzmöglichkeiten gewährleisten.

Diese Aufgabe wird für die Verbindungsvorrichtung dadurch gelöst, dass ein Schlauchabschnitt auf den Schlauchanschlussstutzen klebstofffrei aufgesteckt ist, und dass die Sicherungshülse in zwei - relativ zu einer Mittellängsachse des Verbindungskörpers - zueinander axial beabstandeten Sicherungsstellungen auf dem Verbindungskörper axial sicherbar ist, wobei in einer ersten Sicherungsstellung eine Verdrehbarkeit der Sicherungshülse relativ zu dem Schlauchabschnitt gegeben und in einer zweiten Sicherungsstellung eine Verklemmung der Sicherungshülse auf dem auf den Schlauchanschlussstutzen aufgesteckten Schlauchabschnitt vorgesehen ist. Durch die erfindungsgemäße Lösung wird eine Verbindung zwischen einer Schlauchleitung und dem Verbindungskörper durch eine Klemmung eines entsprechenden Schlauchabschnitts auf dem Schlauchanschlussstutzen des Verbindungskörpers erzielt. Eine Verklebung des Schlauchabschnitts mit einem entsprechenden Schlauchanschlussstutzen eines Verbindungskörpers wird hierdurch vermieden. Aufgrund der lediglich mechanischen Verklemmung des Schlauchabschnitts relativ zum Verbindungskörper und zur Sicherungshülse ist die Auswahl von Materialpaarungen nicht mehr abhängig von der Anpassung an geeignete Klebematerialien und Klebeverfahren. Auch Alterungsprobleme des Klebstoffs, die beim Stand der Technik zu Verklebungs- und Dichtheitsproblemen führen konnten, werden durch die erfindungsgemäße Lösung vermieden. Durch die erfindungsgemäße Lösung können für die Schlauchleitung und den Verbindungskörper und/oder die Sicherungshülse Materialpaarungen gewählt werden, die für eine Verklebung nicht kompatibel wären, insbesondere Polypropylen und ein thermoplastisches Elastomer (TPE). Der Verbindungskörper kann je nach Ausführungsform ein- oder mehrteilig ausgeführt sein. Bei einer bevorzugten Ausführungsform ist in dem Verbindungskörper ein Rückschlagventil integriert. Der Verbindungskörper ist Teil einer Verbindungsvorrichtung, die innerhalb eines medizinischen Fluidleitungssystems eingesetzt werden kann. Der Verbindungskörper kann als Teil einer Tropfkammer vorgesehen sein. Alternativ ist der Verbindungskörper Teil eines medizinischen Infusions- oder Transfusionssystems. In vorteilhafter Weise ist die Verbindungsvorrichtung Teil eines Einweg-Leitungssystems für medizinische Infusionszwecke, das nach einmaliger Benutzung entsorgt wird. In vorteilhafter Weise ist der Verbindungskörper als Verbindungskonnektor ausgeführt, auf dem die Sicherungshülse gehalten ist. In vorteilhafter Weise erfolgt eine Verklemmung und demzufolge Abdichtung des Schlauchabschnitts auf dem Schlauchanschlussstutzen des Verbindungskörpers in einem gemeinsamen Bewegungsvorgang zusammen mit einer Verbindung der Sicherungshülse mit einer weiteren Fluidleitungs- oder -speicherkomponente, insbesondere einem Katheter, einer Spritze oder einem weiteren Verbindungskonnektor. Vorzugsweise ist die Sicherungshülse als lose Luer-Lock-Mutter ausgeführt, so dass eine Verbindung mit einer komplementären Luer-Lock-Komponente zwangsläufig auch eine Verklemmung, Abdichtung und Sicherung des Schlauchabschnitts auf dem Verbindungskörper bewirkt. Beim Anziehen der Luer-Lock-Mutter, d.h. der Sicherungshülse, erfolgt die Überführung der Sicherungshülse aus der ersten in die zweite Sicherungsstellung und dadurch die entsprechende Abdichtung und Klemmung des Schlauchabschnitts radial und axial zwischen der Sicherungshülse und dem Schlauchanschlussstutzen. In der ersten Sicherungsstellung hingegen ist eine Verdrehbarkeit der Sicherungshülse relativ zum Verbindungskörper auch bei bereits aufgestecktem Schlauchabschnitt gegeben. Bei einem Anziehen der Sicherungshülse aus der ersten Sicherungsstellung, wodurch die Sicherungshülse sich neben einer Drehbewegung auch axial in Richtung der zweiten Sicherungsstellung bewegt, erfolgt demzufolge keine Krafteinwirkung oder Drehmomentübertragung auf einen Außenmantel des Schlauchabschnitts, so dass ein Verdrehen der Schlauchleitung vermieden wird.

In Ausgestaltung der Erfindung weisen der Verbindungskörper und die Sicherungshülse zueinander komplementäre Rastprofilierungen auf, die die erste und die zweite Sicherungsstellung der Sicherungshülse auf dem Verbindungskörper definieren, wobei wenigstens eine Rastprofilierung zumindest abschnittsweise radial elastisch nachgiebig ausgeführt ist, um ein hörbares Einrasten der Rastprofilierung in der ersten und/oder der zweiten Sicherungsstellung zu ermöglichen. Die komplementären Rastprofilierungen gewährleisten eine zuverlässige, klar definierte, axial gesicherte Positionierung der Sicherungshülse relativ zum Verbindungskörper. In der ersten Sicherungsstellung ist trotz axialer Sicherung mittels der Rastprofilierungen noch eine Drehbarkeit der Sicherungshülse gewährleistet. Das hörbare Einrasten der wenigstens einen Rastprofilierung verbessert die Handhabung der Verbindungsvorrichtung durch medizinisches Pflegepersonal. In vorteilhafter Weise ist ein hörbares Einrasten nur dann vorgesehen, wenn die wenigstens eine Rastprofilierung die zweite Sicherungsstellung der Sicherungshülse auf dem Verbindungskörper erreicht hat, die gleichzeitig eine Abdichtung und Klemmung des Schlauchabschnitts auf dem Verbindungskörper gewährleistet. Durch das hörbare Einrasten in der zweiten Sicherungsstellung wird dem medizinischen Pflegepersonal akustisch signalisiert, dass eine abgedichtete und geklemmte Sicherungsstellung der Sicherungshülse erreicht ist.

In weiterer Ausgestaltung der Erfindung ist der Verbindungskörper im Bereich des Schlauchanschlussstutzens mit einem - in Aufsteckrichtung eines Schlauchabschnitts gesehen - endseitig angeordneten Anlaufschrägenbereich versehen. Der Schlauchabschnitt wird hierdurch beim Aufstecken stirnendseitig elastisch aufgeweitet, wodurch sich eine verbesserte Klemmung des Schlauchabschnitts auf dem Schlauchanschlussstutzen ergibt. Der Anlaufschrägenbereich kann durch eine umlaufende oder durch mehrere, über den Umfang des Schlauchanschlussstutzens verteilt angeordnete Anlaufschrägenabschnitte gebildet sein.

In weiterer Ausgestaltung der Erfindung ist der Anlaufschrägenbereich als konisch erweiterte Mantelfläche gestaltet. Die konisch erweiterte Mantelfläche bildet eine umlaufende schräge Anlauffläche, die einen Stirnendbereich des Schlauchabschnitts beim Aufstecken des Schlauchabschnitts zwangsläufig entsprechend aufweitet, wodurch eine verbesserte Klemmwirkung des Schlauchabschnitts in diesem Anlaufschrägenbereich erzielt wird.

In weiterer Ausgestaltung der Erfindung ist die Sicherungshülse an einem dem Verbindungsanschluss des Verbindungskörpers zugeordneten Endbereich mit Luer-Lock-Anschlussprofilierungen versehen. Vorzugsweise ist die Sicherungshülse in diesem Endbereich als Luer-Lock-Mutter ausgeführt, so dass die Sicherungshülse mit weiblichen Luer-Lock-Anschlussprofilierungen versehen ist. Die Sicherungshülse ist in der ersten Sicherungsstellung bis hin zur axialen Verlagerung in die zweite Sicherungsstellung sowie auch in der zweiten Sicherungsstellung relativ zum Verbindungskörper drehbar angeordnet. In der zweiten Sicherungsstellung ist jedoch keine Drehbarkeit der Sicherungshülse relativ zum verklemmten Schlauchabschnitt mehr gegeben, so dass ein Weiterdrehen der Sicherungshülse in der zweiten Sicherungsstellung relativ zum Verbindungskörper vorzugsweise nur über begrenzte Drehwinkel erfolgt, die Verwindungen der den Schlauchabschnitt umfassenden Schlauchleitung zumindest weitgehend vermeidet.

In weiterer Ausgestaltung der Erfindung weist die Sicherungshülse an einem dem Schlauchanschlussstutzen zugeordneten anderen Endbereich einen Klemmhülsenabschnitt auf, der zumindest abschnittsweise radial - auf die Mittellängsachse des Verbindungskörpers bezogen - elastisch nachgiebig angeordnet ist und in der zweiten Sicherungsstellung der Sicherungshülse auf den Schlauchabschnitt eine radiale und/oder axiale Klemmwirkung ausübt. In vorteilhafter Weise ist der Klemmhülsenabschnitt einstückig an der Sicherungshülse angeformt. Die radiale elastische Nachgiebigkeit des Klemmhülsenabschnitts ermöglicht ein Zusammenwirken mit einem Außenumfang des Schlauchanschlussstutzens des Verbindungskörpers bei der Klemmung und Deformation des Schlauchabschnitts.

In weiterer Ausgestaltung der Erfindung ist der Klemmhülsenabschnitt durch mehrere, über einen Innenumfang der Sicherungshülse gleichmäßig verteilt angeordnete Klemmlaschen gebildet. Die Klemmlaschen sind einstückig mit der Sicherungshülse verbunden und werden bei Herstellung der Klemmhülse durch entsprechende Werkzeuggestaltung eines Kunststoffspritzgusswerkzeugs erzielt.

In weiterer Ausgestaltung der Erfindung weisen der Klemmhülsenabschnitt und der Schlauchanschlussstutzen zueinander komplementäre Klemmprofilierungen auf, die in der ersten und/oder der zweiten Sicherungsstellung eine radiale und/oder axiale Klemmwirkung auf den Schlauchabschnitt ausüben. Die Klemmprofilierungen sind vorzugsweise einstückig an einem Außenumfang des Schlauchanschlussstutzens bzw. an einem Innenumfang des Klemmhülsenabschnitts vorgesehen und bewirken eine außen- und innenseitige Deformation des Schlauchabschnitts, sobald die erste oder die zweite Sicherungsstellung der Sicherungshülse erreicht ist, sofern der Schlauchabschnitt auf den Schlauchanschlussstutzen aufgesteckt ist.

In weiterer Ausgestaltung der Erfindung ist der Klemmhülsenabschnitt an einem - in Aufsteckrichtung des Schlauchabschnitts gesehen - vorderen Stirnendbereich mit einem Stützschrägenbereich versehen, der zur endseitigen Klemmung des Schlauchabschnitts mit dem Anlaufschrägenbereich des Schlauchanschlussstutzens des Verbindungskörpers zusammenwirkt. Die zueinander komplementären schrägen Anlaufebenen des Stützschrägenbereichs und des Anlaufschrägenbereichs bewirken bei einer Axialbewegung der Sicherungshülse zwangsläufig eine sich mit fortschreitender Axialbewegung erhöhende Klemm- und Deformationswirkung für den Schlauchabschnitt.

In weiterer Ausgestaltung der Erfindung sind die zueinander komplementären Rastprofilierungen der Sicherungshülse und des Verbindungskörpers für die zweite Sicherungsstellung derart gestaltet, dass die Sicherungshülse relativ zu dem Verbindungskörper in Aufsteckrichtung des Schlauchabschnitts begrenzt axial beweglich ist, und dass die Sicherungshülse in entgegengesetzter Axialrichtung axial blockiert ist. Dadurch kann die Sicherungshülse auch nach Erreichen der zweiten Sicherungsstellung noch weiter axial in Richtung der zugeordneten Verbindungskomponente, insbesondere der Fluidleitungs- oder -speicherkomponente, durch entsprechende Verdrehung über das Luer-Lock-Gewinde gezogen werden, wodurch sich eine weitere Erhöhung der Dicht- und Klemmwirkung auf den Schlauchabschnitt ergibt. Ein Lösen in entgegengesetzter Richtung der Sicherungshülse aus der zweiten Sicherungsstellung hingegen ist nicht möglich.

In weiterer Ausgestaltung der Erfindung sind die Rastprofilierungen der Sicherungshülse als mehrere, über einen Innenumfang der Sicherungshülse verteilt angeordnete Rastnasen gestaltet, die begrenzt radial elastisch nachgiebig an der Sicherungshülse angeordnet sind. Die Rastnasen sind einstückig an der Sicherungshülse ausgeformt und sind vorzugsweise an Stegfortsätzen vorgesehen, die achsparallel zu einer Mittellängsachse der Sicherungshülse abragen und eine radiale, elastische Nachgiebigkeit der Rastnasen gewährleisten.

In weiterer Ausgestaltung der Erfindung ist die Sicherungshülse im Bereich eines Außenmantels mit Griffprofilierungen zum manuellen Handhaben der Sicherungshülse versehen. Dadurch ist ein einfaches und haptisch gut erfassbares Verdrehen der Sicherungshülse gewährleistet.

Für das medizinische Fluidleitungssystem der eingangs genannten Art wird die der Erfindung zugrundeliegende Aufgabe dadurch gelöst, dass die Verbindungsvorrichtung gemäß zumindest einer der zuvor beschriebenen Ausführungen oder Ausgestaltungen ausgeführt ist. Vorzugsweise ist das medizinische Fluidleitungssystem als Schlauchleitungssystem eines medizinischen Infusionssystems vorgesehen.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt in einer Seitenansicht eine Ausführungsform einer erfindungsgemäßen Verbindungsvorrichtung für ein medizinisches Fluidleitungssystem,
- Fig. 2: die Verbindungsvorrichtung nach Fig. 1 in einer Schnittdarstellung längs der Schnittlinie II-II in Fig. 1,
- Fig. 3: die Verbindungsvorrichtung nach Fig. 1 in einer gegenüber der Darstellung in Fig. 1 axial versetzten zweiten Sicherungsstellung einer Sicherungshülse,
- Fig. 4: die Verbindungsvorrichtung nach Fig. 3 in einem Längsschnitt entlang der Schnittlinie IV-IV in Fig. 3,
- Fig. 5: in vergrößerter Darstellung einen Ausschnitt V der Verbindungsvorrichtung nach Fig. 2,
- Fig. 6: in vergrößerter Darstellung einen Ausschnitt VI der Verbindungsvorrichtung gemäß Fig. 4,
- Fig. 7: in weiter vergrößerter Darstellung einen Ausschnitt VII aus Fig. 6 und
- Fig. 8: in vergrößerter Darstellung einen Ausschnitt VIII aus Fig. 6.

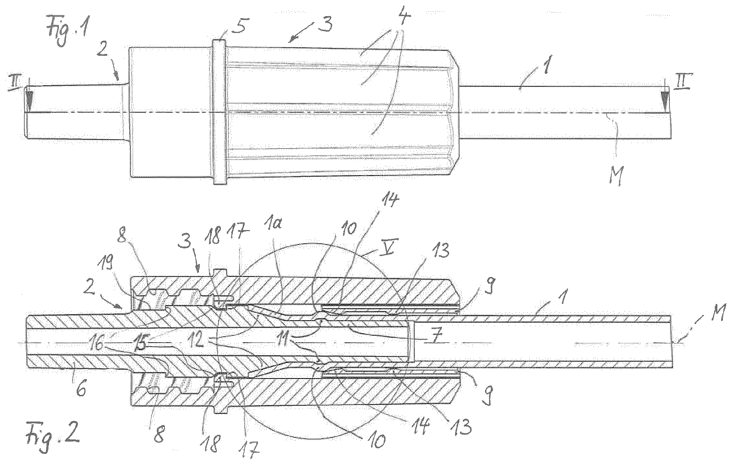

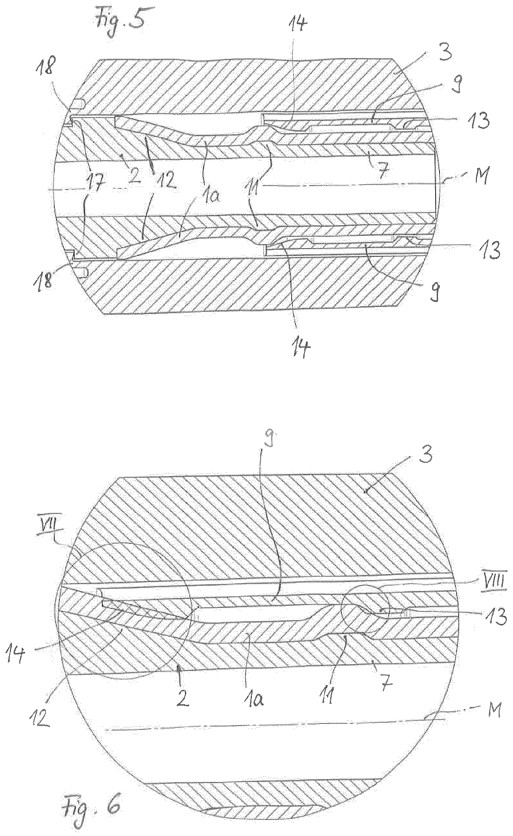

Eine Verbindungsvorrichtung nach den Fig. 1 bis 8 ist Teil eines medizinischen Fluidleitungssystems in Form eines Infusionssystems. Die Verbindungsvorrichtung dient dazu, eine Schlauchleitung 1 mit einer medizinischen Fluidleitungs- oder -speicherkomponente des medizinischen Fluidleitungssystems zu verbinden. Die Schlauchleitung 1 weist einen endseitigen Schlauchabschnitt 1a auf, der eine gegenüber der Schlauchleitung 1 reduzierte Wandungsdicke aufweist und demzufolge eine erhöhte Flexibilität besitzt. Die Schlauchleitung 1 einschließlich des Schlauchabschnitts 1a ist aus einem elastisch flexiblen Kunststoffmaterial, vorliegend aus einem thermoplastischen Elastomer, vorzugsweise Silikon, hergestellt.

Die Verbindungsvorrichtung weist zudem einen einteiligen Verbindungskörper 2 aus einem thermoplastischen Kunststoff, vorliegend aus Polypropylen, auf. Der Verbindungskörper 2 ist von einer ringförmigen Sicherungshülse 3 koaxial - auf eine Mittellängsachse M des Verbindungskörpers bezogen - umgeben. Die Sicherungshülse 3 stellt ein zu dem Verbindungskörper 2 und der Schlauchleitung 1 getrenntes Kunststoffbauteil dar, das ebenfalls einteilig ausgeführt ist.

Der Verbindungskörper 2 weist beim dargestellten Ausführungsbeispiel einen koaxial zur Mittellängsachse M erstreckten, zylindrischen Durchtrittskanal auf, der zu gegenüberliegenden Stirnendbereichen des Verbindungskörpers 2 hin offen ist. Bei einem nicht dargestellten Ausführungsbeispiel der Erfindung ist in dem Verbindungskörper 2 zusätzlich ein Rückschlagventil integriert. Der nicht dargestellte Verbindungskörper wird durch zwei miteinander verbundene und das Rückschlagventil zwischen diesen einschließende Verbindungsbauteile gebildet.

Der Verbindungskörper 2 weist auf einer der Schlauchleitung 1 zugewandten Seite einen Schlauchanschlussstutzen 7 auf, auf den der Schlauchabschnitt 1a bei der Montage der Schlauchleitung 1 axial aufgezogen wird. Im Bereich seiner gegenüberliegenden Seite ist der Verbindungskörper 2 mit einem Verbindungsanschluss 6 versehen, der als geringfügig zu einem Stirnendbereich hin konisch verjüngte Steckspitze ausgeführt ist.

Die Sicherungshülse 3 ist an ihrem Außenumfang zum einen mit Griffprofilierungen 4 für eine einfache manuelle Handhabung der Sicherungshülse 3 versehen. Zum anderen weist die Sicherungshülse 3 an ihrem Außenumfang einen radial nach außen ragenden Ringbund 5 auf, der die Griffprofilierungen 4 axial begrenzt. Der Ringbund 5 dient ebenfalls zur einfachen manuellen Handhabung der Sicherungshülse 3.

Die Sicherungshülse 3 ist auf ihrer dem Verbindungsanschluss 6 zugewandten Seite mit einem weiblichen Luer-Lock-Gewinde versehen, das als Luer-Lock-Anschlussprofilierung 8 dient. Der so gebildete Abschnitt der Sicherungshülse 3 bildet demzufolge eine Luer-Lock-Mutter. Zu einem gegenüberliegenden Stirnende hin weist die Sicherungshülse 3 an ihrem Innenumfang einen ringförmigen Klemmhülsenabschnitt 9 auf, der durch mehrere parallel zueinander verlaufende, gleichmäßig über den Innenumfang der Sicherungshülse 3 verteilt angeordnete Klemmlaschen gebildet ist. Die Klemmlaschen erstrecken sich parallel zur Mittellängsachse M längs des Innenumfangs der Sicherungshülse 3. Die Klemmlaschen 9 sind in Umfangsrichtung einstückig mit der Sicherungshülse 3 verbunden. Radial nach außen befindet sich zwischen dem Innenumfang der Sicherungshülse 3 und der jeweiligen Klemmlasche jedoch ein Längsspalt, der die radial elastische Nachgiebigkeit der jeweiligen Klemmlasche und damit des Klemmhülsenabschnitts 9 gewährleistet. Die Sicherungshülse 3 ist ebenfalls aus einem thermoplastischen Kunststoffmaterial hergestellt, vorzugsweise aus dem gleichen Material wie der Verbindungskörper 2. Der Klemmhülsenabschnitt 9 weist einen Innendurchmesser auf, der größer ist als ein Außendurchmesser der Schlauchleitung 1. Der Klemmhülsenabschnitt 9 ist mit Klemmprofilierungen 13, 14 versehen, die axial zueinander beabstandet sind und noppenartig radial zum Schlauchabschnitt 1a bzw. zur Schlauchleitung 1 nach innen abragen. Ein Innendurchmesser des Klemmhülsenabschnitts 9 im Bereich dieser Klemmprofilierungen 13 ist geringfügig größer oder gleich groß wie ein Außendurchmesser der Schlauchleitung 1 und des Schlauchabschnitts 1a, so dass die Sicherungshülse 3 auf der Schlauchleitung 1 und dem Schlauchabschnitt 1a im Wesentlichen frei axial verschoben oder in Umfangsrichtung verdreht werden kann, ohne dass sich eine Deformation und demzufolge ein Widerstand der Schlauchleitung 1 oder des Schlauchabschnitts 1a einstellt.

Die Klemmprofilierungen 14 des Klemmhülsenabschnitts 9, die an einem den Luer-Lock-Anschlussprofilierungen 8 zugewandten Stirnendbereich des Klemmhülsenabschnitts 9 vorgesehen sind, weisen jeweils eine sich zu einem Stirnende hin erweiternde Anlaufschräge auf.

Der Verbindungskörper 2 weist im Bereich des Schlauchanschlussstutzens 7 eine komplementär an einem Außenmantel angeformte und radial nach außen ragende Klemmprofilierung 11 auf, die als höckerförmiger Ringsteg ausgeführt ist. Zu der Klemmprofilierung 11 axial in Richtung des Verbindungsanschlusses 6 beabstandet weist der Verbindungskörper 2 an einem Au-ßenumfang des Schlauchanschlussstutzens 7 zudem einen Anlaufschrägenbereich in Form einer konisch erweiterten Mantelfläche 12 auf, die von einem zylindrischen Außenumfang des Schlauchanschlussstutzens 7 ausgehend in Richtung des Verbindungsanschlusses 6 erweitert ist. An die konisch erweiterte Mantelfläche 12 axial anschließend ist ein zylindrischer Außenmantelbereich des Verbindungskörpers 2 vorgesehen, der mit einer ringnutförmigen Rastprofilierung 15, 17 versehen ist. Die Rastprofilierung 15, 17 weist eine stufenartig gestaltete Rastschulter 17 sowie eine zum Verbindungsanschluss 6 hin erstreckte, konisch schräg verlaufende Gleitschulter 15 auf, die sich in die zylindrische Außenmantelfläche des Verbindungskörpers 2 fortsetzt. Axial in Richtung des Verbindungsanschlusses 6 beabstandet ist eine weitere, als radiale Ringschulter gestaltete Rastprofilierung 16 vorgesehen, die in einen zylindrischen Außenmantelbereich 19 mit gegenüber der Außenmantelfläche verringertem Außendurchmesser übergeht. Von dem zylindrischen Außenmantelbereich 19 aus erstreckt sich die konisch verjüngte Steckspitze, die den Verbindungsanschluss 6 des Verbindungskörpers 2 bildet.

Die Sicherungshülse 3 ist an ihrem Innenumfang axial anschließend an die Luer-Lock-Anschlussprofilierung 8 mit elastisch nachgiebigen Rastprofilierungen 18 versehen, die einstückig an der Sicherungshülse 3 angeformt sind und durch mehrere, über den Innenumfang der Sicherungshülse 3 verteilt angeordnete Rastnasen 18 gebildet sind. Die Rastnasen 18 ragen radial nach innen und sind an radial elastisch nachgiebigen Stegen angeordnet.

Die Klemmprofilierungen 14 bilden einen Stützschrägenbereich, der komplementär zu dem Anlaufschrägenbereich 12 des Verbindungskörpers 2 ausgeführt ist und eine axiale und radiale Klemmung des Schlauchabschnitts 1a auf dem Schlauchanschlussstutzen 7 des Verbindungskörpers 2 bewirkt.

Für eine Montage der Verbindungsvorrichtung und des entsprechenden medizinischen Fluidleitungssystems wird in einem ersten Schritt die Sicherungshülse 3 axial von einer Stirnseite her auf die Schlauchleitung 1 aufgeschoben. Anschließend wird die Schlauchleitung 1 mit ihrem Schlauchabschnitt 1a axial auf den Schlauchanschlussstutzen 7 des Verbindungskörpers 2 aufgesteckt, vorzugsweise durch Aufziehen oder Aufschieben des Schlauchabschnitts 1a. Der Schlauchabschnitt 1a wird so weit auf den Schlauchanschlussstutzen 7 aufgeschoben, bis ein Stirnendbereich des Schlauchabschnitts 1a an dem Anlaufschrägenbereich 12 entlanggleitet und aufgeweitet wird, und bis ein Stirnrand des Schlauchabschnitts 1a an einer den Anlaufschrägenbereich 12 begrenzenden, nicht näher bezeichneten Ringschulter zur Anlage kommt.

Anschließend wird die Sicherungshülse 3 von der Schlauchleitung 1 aus in Richtung des Verbindungskörpers 2 axial verschoben, bis die Rastnasen 18 in der ringnutförmigen Rastprofilierung 15, 17 hörbar einrasten. In dieser Stellung ist die erste Sicherungsstellung der Sicherungshülse 3 relativ zum Verbindungskörper 2 erreicht. Die Sicherungshülse 3 ist aufgrund der Lagerung der Rastnasen 18 in der ringnutförmigen Rastprofilierung 15, 17 zum einen auf dem Verbindungskörper 2 axial gesichert und zum anderen relativ zu dem Verbindungskörper 2 drehbar gelagert. Der Klemmhülsenabschnitt ist teilweise auf den Schlauchabschnitt 1a axial aufgeschoben. In der ersten Sicherungsstellung der Sicherungshülse 3, die anhand der Fig. 2 erkennbar ist, liegt der Stützschrägenbereich der Klemmprofilierung 14 des Klemmhülsenabschnitts 9 an einem aufgewölbten Schlauchbereich 10 des Schlauchabschnitts 1a an und bewirkt eine gewisse axiale und radiale Klemmung des Schlauchabschnitts 1a zusätzlich zu der Klemmung, die der Schlauchabschnitt 1a aufgrund seiner elastischen Aufweitung auf dem Au-ßenumfang des Schlauchanschlussstutzens 7 von sich heraus bereits erfahren hat.

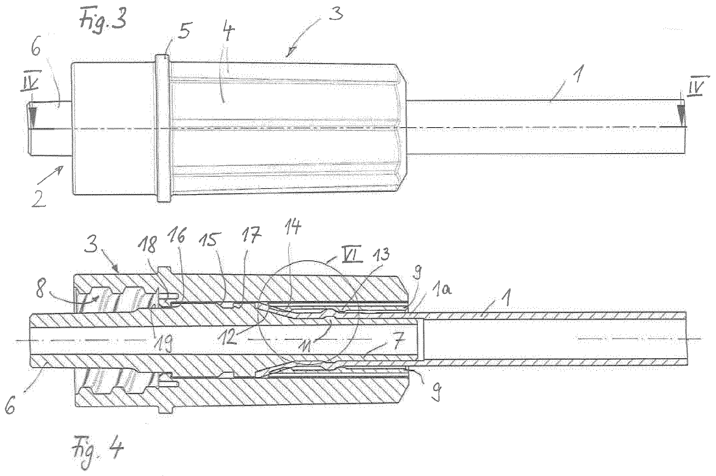

Diese erste Sicherungsstellung der Sicherungshülse 3 entspricht einer Bereitstellungsposition der Verbindungsvorrichtung, bevor diese mit einem anderen Verbindungskonnektor einer Fluidleitungs- oder -speicherkomponente im medizinischen Betrieb verbunden wird. Sobald die Verbindungsvorrichtung in der ersten Sicherungsstellung gemäß Fig. 2 nun einem komplementären Verbindungsbauteil des medizinischen Fluidsystems, insbesondere des medizinischen Infusionssystems, zugeführt wird, kommen der Verbindungsanschluss 6 und die Luer-Lock-Anschlussprofilierungen 8 der Verbindungsvorrichtung in nicht näher dargestellter Weise mit komplementären Luer-Lock-Anschlussprofilierungen sowie einer Steckaufnahme des Verbindungsbauteils in Kontakt. Die komplementären Luer-Lock-Anschlussprofilierungen des nicht dargestellten Verbindungsbauteils sind als männliche Luer-Lock-Anschlussprofilierungen ausgeführt, um eine Konnektierung zwischen dem Verbindungsbauteil und der Sicherungshülse 3 herstellen zu können. Sobald die Steckaufnahme des Verbindungsbauteils und die Steckspitze des Verbindungsanschlusses 6 des Verbindungskörpers 2 so weit zusammengesteckt sind, dass eine axiale Vorfixierung erreicht ist, wird die Sicherungshülse 3 verdreht, wodurch sich zwangsläufig die Luer-Lock-Mutter der Sicherungshülse 3 auf den männlichen Luer-Lock-Anschlussprofilierungen des Verbindungsbauteils festziehen. Da der Verbindungskörper 2 selbst bereits durch das Verbindungsbauteil axial blockiert ist, gleitet die Sicherungshülse 3 zusätzlich zu ihrer Drehbewegung zwangsläufig axial auf dem Außenmantelbereich des Verbindungskörpers 2 entlang, wobei die Rastnasen 18 längs der Anlaufschulter 15 aus der Rastprofilierung 15, 17 herausgleiten. Sobald die Rastnasen 18 über die Rastprofilierung 16 hinaus axial verlagert sind, rasten die Rastnasen 18 erneut hörbar an der durch die radiale Ringschulter gebildeten Rastprofilierung 16 ein. Dann ist die zweite Sicherungsstellung gemäß Fig. 4 erreicht.

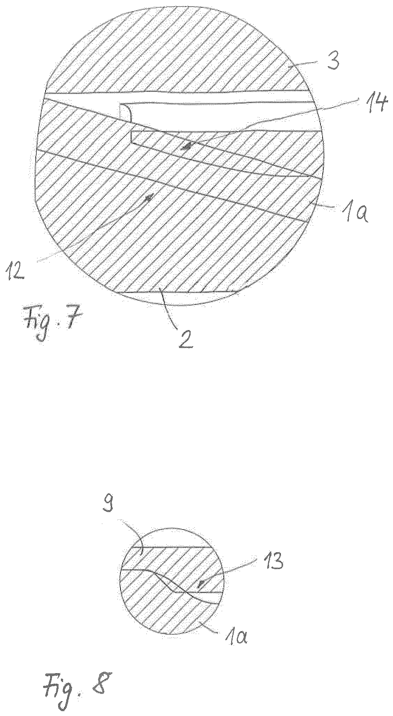

Zusammen mit der axialen Verlagerung der Sicherungshülse 3 hat sich zwangsläufig bei der Überführung aus der ersten Sicherungsstellung in die zweite Sicherungsstellung auch der Klemmhülsenabschnitt 9 axial längs des Schlauchanschlussstutzens 7 verlagert, wodurch der Stützschrägenabschnitt der Klemmprofilierungen 14 an dem auf dem Anlaufschrägenbereich 12 erweiterten Stirnendbereich des Schlauchabschnitts 1a aufläuft und hierdurch elastisch nach außen deformiert wird. Ergänzend läuft die Klemmprofilierung 13 auf die höckerartige Auswölbung 10 des Schlauchabschnitts 1a im Bereich der am Außenumfang des Schlauchanschlussstutzens 7 vorgesehenen Klemmprofilierung 11 auf und bewirkt hier eine zusätzliche Deformation sowie eine zusätzliche radiale und axiale Klemmung des Schlauchabschnitts 1a. Die Positionierung des Stützschrägenbereichs der Klemmprofilierung 14 und der Klemmprofilierung 13 in dieser zweiten Sicherungsstellung der Sicherungshülse 3 relativ zum Schlauchabschnitt 1a und relativ zum Verbindungskörper 2 ist anhand der Fig. 7 und 8 vergrößert dargestellt.

Da an den radialen Ringschulterabschnitt des Verbindungskörpers 2, der die Rastprofilierung 16 für die zweite Sicherungsstellung bildet, die zylindrische Außenmantelfläche 19 mit gegenüber dem Außenmantelbereich des Verbindungskörpers reduziertem Durchmesser anschließt, kann die Sicherungshülse 3 auch in dieser zweiten Sicherungsstellung noch axial in Richtung zum Stirnende der Steckspitze, d.h. des Verbindungsanschlusses 6, weiter verlagert werden. Das Erreichen der zweiten Sicherungsstellung jedenfalls ist für eine Bedienperson bei Handhabung der Sicherungshülse 3 ebenfalls durch ein hörbares Einrasten erkennbar. Falls die Sicherungshülse 3 mit ihren Luer-Lock-Anschlussprofilierungen 8 noch weiter in Verschlussrichtung gedreht wird, kann die Sicherungshülse 3 sich noch weiter axial in Richtung des Verbindungsanschlusses 6 bewegen, wodurch die Klemmwirkung des Stützschrägenbereichs 14 und der Klemmprofilierungen 13 auf den Schlauchabschnitt 1a weiter erhöht wird.

Ein Lösen der Sicherungshülse 3 von dem Verbindungskörper 2 nach Erreichen der zweiten Sicherungsstellung ist nicht ohne weiteres und insbesondere nicht ohne Beschädigung der Sicherungshülse 3 möglich. Dies ist auch nicht notwendig, da die Verbindungsvorrichtung und das entsprechende Schlauchleitungssystem zur Einmalbenutzung als Einwegbesteck ausgeführt sind.

## Patentansprüche

1. Verbindungsvorrichtung für ein medizinisches Fluidleitungssystem mit einem Verbindungskörper (2), der einen Schlauchanschlussstutzen (7) sowie einen Verbindungsanschluss (6) zur lösbaren Befestigung einer weiteren Fluidleitungs- oder - speicher¬komponente aufweist, wobei der Verbindungskörper (2) von einer Sicherungshülse (3) koaxial umgeben ist und wobei in betriebsfertigem Zustand eine Schlauchleitung (1) mit dem Schlauchanschlussstutzen (7) verbunden ist, **dadurch gekennzeichnet, dass** ein Schlauchabschnitt der Schlauchleitung (1) auf den Schlauchanschlussstutzen (7) klebstofffrei aufgesteckt ist, und dass die Sicherungshülse (3) in zwei - relativ zu einer Mittellängsachse (M) des Verbindungskörpers (2) - zueinander axial beabstandeten Sicherungsstellungen auf dem Verbindungskörper (2) axial sicherbar ist, wobei in einer ersten Sicherungsstellung eine Verdrehbarkeit der Sicherungshülse (3) relativ zu dem Schlauchabschnitt gegeben ist und in einer zweiten Sicherungsstellung eine Verklemmung der Sicherungshülse (3) auf dem auf den Schlauchanschlussstutzen (7) aufgesteckten Schlauchabschnitt (1a) vorgesehen ist, und dass der Verbindungskörper (2) und die Sicherungshülse (3) zueinander komplementäre Rastprofilierungen (15 bis 18) aufweisen, die die erste und die zweite Sicherungsstellung der Sicherungshülse (3) auf dem Verbindungskörper (2) definieren, wobei wenigstens eine Rastprofilierung (18) zumindest abschnittsweise radial elastisch nachgiebig ausgeführt ist, um ein hörbares Einrasten der Rastprofilierung (18) in der ersten und/oder der zweiten Sicherungsstellung zu ermöglichen.

2. Verbindungsvorrichtung nach Anspruch 1, **dadurch gekennzeichnet, dass** der Verbindungskörper (2) im Bereich des Schlauchanschlussstutzens (7) mit einem - in Aufsteckrichtung eines Schlauchabschnitts (1a) gesehen - endseitig angeordneten Anlaufschrägenbereich (12) versehen ist.

3. Verbindungsvorrichtung nach Anspruch 2, **dadurch gekennzeichnet, dass** der Anlaufschrägenbereich (12) als konisch erweiterte Mantelfläche gestaltet ist

4. Verbindungsvorrichtung nach Anspruch 1, **dadurch gekennzeichnet, dass** die Sicherungshülse (3) an einem dem Verbindungsanschluss (6) des Verbindungskörpers (2) zugeordneten Endbereich mit Luer-Lock-Anschlussprofilierungen (8) versehen ist.

5. Verbindungsvorrichtung nach einem der vorhergehenden Ansprüche, **dadurch gekennzeichnet, dass** die Sicherungshülse (3) an einem dem Schlauchanschlussstutzen (7) zugeordneten anderen Endbereich einen Klemmhülsenabschnitt (9) aufweist, der zumindest abschnittsweise radial - auf die Mittellängsachse (M) des Verbindungskörpers (2) bezogen - elastisch nachgiebig angeordnet ist und in der zweiten Sicherungsstellung der Sicherungshülse (3) auf den Schlauchabschnitt (1a) eine radiale und/oder axiale Klemmwirkung ausübt.

6. Verbindungsvorrichtung nach Anspruch 5, **dadurch gekennzeichnet, dass** der Klemmhülsenabschnitt (9) durch mehrere, über einen Innenumfang der Sicherungshülse (3) gleichmäßig verteilt angeordnete Klemmlaschen gebildet ist.

7. Verbindungsvorrichtung nach Anspruch 5 oder 6, **dadurch gekennzeichnet, dass** der Klemmhülsenabschnitt (9) und der Schlauchanschlussstutzen (7) zueinander komplementäre Klemmprofilierungen (11, 13) aufweisen, die in der ersten und/oder der zweiten Sicherungsstellung eine radiale und/oder axiale Klemmwirkung auf den Schlauchabschnitt (1a) ausüben.

8. Verbindungsvorrichtung nach Anspruch 7, **dadurch gekennzeichnet, dass** der Klemmhülsenabschnitt (9) an einem - in Aufsteckrichtung des Schlauchabschnitts gesehen - vorderen Stirnendbereich mit einem Stützschrägenbereich (14) versehen ist, der zur endseitigen Klemmung des Schlauchabschnitts (1a) mit dem Anlaufschrägenbereich (12) des Schlauchanschlussstutzens (7) des Verbindungskörpers (2) zusammenwirkt.

9. Verbindungsvorrichtung nach einem der vorhergehenden Ansprüche, **dadurch gekennzeichnet, dass** die zueinander komplementären Rastprofilierungen (16, 18) der Sicherungshülse (3) und des Verbindungskörpers (2) für die zweite Sicherungsstellung derart gestaltet sind, dass die Sicherungshülse (3) relativ zu dem Verbindungskörper (2) in Aufsteckrichtung des Schlauchabschnitts (1a) begrenzt axial beweglich ist, und dass die Sicherungshülse (3) in entgegengesetzter Axialrichtung axial blockiert ist.

10. Verbindungsvorrichtung nach einem der vorhergehenden Ansprüche, **dadurch gekennzeichnet, dass** die Rastprofilierungen (18) der Sicherungshülse (3) als mehrere, über einen Innenumfang der Sicherungshülse (3) verteilt angeordnete Rastnasen (18) gestaltet sind, die begrenzt radial elastisch nachgiebig an der Sicherungshülse (3) angeordnet sind.

11. Verbindungsvorrichtung nach einem der vorhergehenden Ansprüche, **dadurch gekennzeichnet, dass** die Sicherungshülse (3) im Bereich eines Außenmantels mit Griffprofilierungen (4) zum manuellen Handhaben der Sicherungshülse (3) versehen ist.

12. Medizinisches Fluidleitungssystem mit einer Schlauchleitung (1, 1a) sowie mit einer Verbindungsvorrichtung zur Befestigung einer weiteren Fluidleitungs- oder -speicherkomponente, wobei die Verbindungsvorrichtung einen Verbindungskörper (2) aufweist, mit der ein Schlauchabschnitt (1a) der Schlauchleitung (1) verbunden ist, **dadurch gekennzeichnet, dass** die Verbindungsvorrichtung gemäß wenigstens einem der vorhergehenden Ansprüche ausgeführt ist.

## Claims

1. Connection device for a medical fluid conduit system, having a connection body (2) which has a hose adapter (7) and a connector piece (6) for releasably fastening a further fluid conduit component or fluid storage component, wherein the connection body (2) is coaxially surrounded by a securing sleeve (3), and wherein in an operationally ready state a hose conduit (1) is connected to the hose adapter (7), **characterized in that** a hose portion of the hose conduit (1) is plug-fitted in an adhesive-free manner onto the hose adapter (7), and **in that** the securing sleeve (3) is axially securable on the connection body (2) in two securing positions that are axially mutually spaced apart relative to a central longitudinal axis (M) of the connection body (2), wherein rotatability of the securing sleeve (3) relative to the hose portion is provided in a first securing position, and jamming of the securing sleeve (3) on the hose portion (1a) that is plug-fitted onto the hose adapter (7) is provided in a second securing position, and **in that** the connection body (2) and the securing sleeve (3) have mutually complementary profiled latching features (15 to 18) which define the first and the second securing position of the securing sleeve (3) on the connection body (2), wherein at least one profiled latching feature (18) at least in portions is embodied so as to be resilient in a radially elastic manner in order for an audible latching of the profiled latching feature (18) to be enabled in the first and/or the second securing position.

2. Connection device according to claim 1, **characterized in that** the connection body (2) in the region of the hose adapter (7) is provided with a run-up ramp region (12) that when viewed in the plug-fitting direction of a hose portion (1a) is disposed on the end side.

3. Connection device according to claim 2, **characterized in that** the run-up ramp region (12) is designed as a conically widening surface area.

4. Connection device according to claim 1, **characterized in that** the securing sleeve (3) on an end region that is assigned to the connector piece (6) of the connection body (2) is provided with profiled Luer lock connectors (8).

5. Connection device according to any of the preceding claims, **characterized in that** the securing sleeve (3) on another end region that is assigned to the hose adapter (7) has a clamping sleeve portion (9) which in relation to the central longitudinal axis (M) of the connection body (2) is disposed so as to be at least partially radially elastically resilient and in the second securing position of the securing sleeve (3) exerts a radial and/or axial clamping effect on the hose portion (1a).

6. Connection device according to claim 5, **characterized in that** the clamping sleeve portion (9) is formed by a plurality of clamping lugs that are disposed so as to be uniformly distributed across an internal circumference of the securing sleeve (3).

7. Connection device according to claim 5 or 6, **characterized in that** the clamping sleeve portion (9) and the hose adapter (7) have mutually complementary profiled clamping features (11, 13) which in the first and/or the second securing position exert a radial and/or axial clamping effect on the hose portion (1a).

8. Connection device according to claim 7, **characterized in that** the clamping sleeve portion (9) when viewed in the plug-fitting direction of the hose portion is provided on a forward front end region with a support ramp region (14) which for clamping the hose portion (1a) at the end side interacts with the run-up ramp region (12) of the hose adapter (7) of the connection body (2).

9. Connection device according to any of the preceding claims, **characterized in that** the mutually complementary profiled latching features (16, 18) of the securing sleeve (3) and of the connection body (2) for the second securing position are designed in such a manner that the securing sleeve (3) in the plug-fitting direction of the hose portion (1a) is axially movable in a limited manner relative to the connection body (2), and **in that** the securing sleeve (3) in the opposite axial direction is axially blocked.

10. Connection device according to any of the preceding claims, **characterized in that** the profiled latching features (18) of the securing sleeve (3) are designed as a plurality of latching cams (18) that are disposed so as to be distributed across an internal circumference of the securing sleeve (3), said latching cams (18) being disposed on the securing sleeve (3) so as to be elastically resilient in a radial and limited manner.

11. Connection device according to any of the preceding claims, **characterized in that** the securing sleeve (3) in the region of an external jacket is provided with profiled grip features (4) for manually handling the securing sleeve (3).

12. Medical fluid conduit system having a hose conduit (1, 1a) and a connection device for fastening a further fluid conduit component or fluid storage component, wherein the connection device has a connection body (2) to which a hose portion (1a) of the hose conduit (1) is connected, **characterized in that** the connection device is embodied according to at least one of the preceding claims.

## Revendications

1. Dispositif de raccordement pour un système de ligne de fluide médical avec un corps de raccordement (2), qui présente une tubulure de raccordement de tuyau flexible (7) ainsi qu'un raccord de raccordement (6) pour la fixation détachable d'un autre composant de ligne ou de réservoir de fluide, dans lequel le corps de raccordement (2) est entouré de façon coaxiale par une douille de blocage (3) et dans lequel dans l'état prêt à fonctionner une ligne flexible (1) est raccordée à la tubulure de raccordement de tuyau flexible (7), **caractérisé en ce qu'**une partie de tuyau flexible de la ligne flexible (1) est engagée sans colle sur la tubulure de raccordement de tuyau flexible (7), et **en ce que** la douille de blocage (3) peut être bloquée axialement sur le corps de raccordement (2) dans deux positions de blocage axialement espacées l'une de l'autre - par rapport à un axe longitudinal central (M) du corps de raccordement (2) -, dans lequel dans une première position de blocage il existe une capacité de rotation de la douille de blocage (3) par rapport à la partie de tuyau flexible et dans une deuxième position de blocage il est prévu un serrage de la douille de blocage (3) sur la partie de tuyau flexible (1a) engagée sur la tubulure de raccordement de tuyau flexible (7), et **en ce que** le corps de raccordement (2) et la douille de blocage (3) présentent des profilages d'encliquetage complémentaires l'un à l'autre (15 à 18), qui définissent la première et la deuxième position de blocage de la douille de blocage (3) sur le corps de raccordement (2), dans lequel au moins un profilage d'encliquetage (18) est réalisé au moins localement sous forme élastiquement déformable radialement, afin de permettre un encliquetage audible du profilage d'encliquetage (18) dans la première et/ou dans la deuxième position de blocage.

2. Dispositif de raccordement selon la revendication 1, **caractérisé en ce que** le corps de raccordement (2) est doté dans la région de la tubulure de raccordement de tuyau flexible (7) d'une zone oblique d'accès (12) disposée en extrémité - considérée dans la direction d'engagement d'une partie de tuyau flexible (1a).

3. Dispositif de raccordement selon la revendication 2, **caractérisé en ce que** la zone oblique d'accès (12) est formée par une surface latérale évasée en cône.

4. Dispositif de raccordement selon la revendication 1, **caractérisé en ce que** la douille de blocage (3) est dotée de profilages de raccordement Luer-Lock (8) sur une région d'extrémité associée au accord de raccordement (6) du corps de raccordement (2) .

5. Dispositif de raccordement selon l'une quelconque des revendications précédentes, **caractérisé en ce que** la douille de blocage (3) présente à une autre région d'extrémité associée à la tubulure de raccordement de tuyau flexible (7) une partie de douille de serrage (9), qui est disposée au moins localement de façon élastiquement déformable radialement - par rapport à l'axe longitudinal central (M) du corps de raccordement (2) - et qui exerce dans la deuxième position de blocage de la douille de blocage (3) un effet de serrage radial et/ou axial sur la partie de tuyau flexible (1a).

6. Dispositif de raccordement selon la revendication 5, **caractérisé en ce que** la partie de douille de serrage (9) est formée par plusieurs pattes de serrage disposées de façon uniformément répartie sur une périphérie intérieure de la douille de blocage (3).

7. Dispositif de raccordement selon la revendication 5 ou 6, **caractérisé en ce que** la partie de douille de serrage (9) et la tubulure de raccordement de tuyau flexible (7) présentent des profilages de serrage (11, 13) complémentaires l'un à l'autre, qui dans la première et/ou dans la deuxième position de blocage exercent un effet de serrage radial et/ou axial sur la partie de tuyau flexible (1a).

8. Dispositif de raccordement selon la revendication 7, **caractérisé en ce que** la partie de douille de serrage (9) est dotée à une région d'extrémité frontale avant - considérée dans la direction d'engagement de la partie de tuyau flexible - d'une zone oblique de soutien (14), qui coopère avec la zone oblique d'accès (12) de la tubulure de raccordement de tuyau flexible (7) du corps de raccordement (2) pour le serrage d'extrémité de la partie de tuyau flexible (1a).

9. Dispositif de raccordement selon l'une quelconque des revendications précédentes, **caractérisé en ce que** les profilages d'encliquetage complémentaires l'un à l'autre (16, 18) de la douille de blocage (3) et du corps de raccordement (2) pour la deuxième position de blocage sont réalisés de telle manière que la douille de blocage (3) soit mobile de façon limitée axialement par rapport au corps de raccordement (2) dans la direction d'engagement de la partie de tuyau flexible (la), et **en ce que** la douille de blocage (3) est bloquée axialement dans la direction axiale opposée.

10. Dispositif de raccordement selon l'une quelconque des revendications précédentes, **caractérisé en ce que** les profilages d'encliquetage (18) de la douille de blocage (3) sont réalisés sous la forme de plusieurs ergots d'encliquetage (18) disposés de façon répartie sur une périphérie intérieure de la douille de blocage (3), qui sont disposés de façon élastiquement déformable radialement de façon limitée sur la douille de blocage (3) .

11. Dispositif de raccordement selon l'une quelconque des revendications précédentes, **caractérisé en ce que** la douille de blocage (3) est dotée dans la région d'une surface latérale extérieure de profilages de saisie (4) pour la manipulation manuelle de la douille de blocage (3) .

12. Système de ligne de fluide médical avec une ligne flexible (1, 1a) ainsi qu'avec un dispositif de raccordement pour la fixation d'un autre composant de ligne ou de réservoir de fluide, dans lequel le dispositif de raccordement présente un corps de raccordement (2), auquel une partie de tuyau flexible (1a) de la ligne flexible (1) est raccordée, **caractérisé en ce que** le dispositif de raccordement est réalisé selon au moins une des revendications précédentes.
